# EUROPEAN PATENT SPECIFICATION

(11) **EP 1 962 826 B1**
(45) Date of publication and mention of the grant of the patent: **16.11.2011**
(21) Application number: 06842216.1
(22) Date of filing: 04.12.2006
(51) Int. Cl.: A61K 31/205, A61K 31/353, A23L 1/29, A61K 36/185, A61P 3/04, A61P 5/48

(54) **METHOD FOR WEIGHT REDUCTION**
VERFAHREN ZUR GEWICHTSREDUKTION
PROCEDE DE REDUCTION DU POIDS

(30) Priority: 05.12.2005 US 741889 P
(43) Date of publication of application: 03.09.2008
(73) Proprietor: Energy4Life GmbH, 4313 Möhlin (CH)
(72) Inventor: ROHNER, Markus, CH-4313 Möhlin (CH)
(74) Representative: Schöllhorn, Andreas
(86) International application number: PCT/IB2006/003455
(87) International publication number: WO 2007/066192

(56) References cited:
- WO-A-99/07388
- WO-A1-2005/034650
- ALBANESE C V ET AL: "FAT MASS LOSS IN OBESE SUBJECTS TREATED WITH CARNITINE: A WHOLE BODY DXA EVALUATION" JOURNAL OF BONE AND MINERAL RESEARCH, NEW YORK, NY, US, vol. 20, no. 9 SUPPL 1, 23 September 2005 (2005-09-23), page S218, XP009085780 ISSN: 0884-0431
- LOFGREN INGRID E ET AL: "WEIGHT LOSS FAVORABLY MODIFIES ANTHROPOMETRICS AND REVERSES THE METABOLIC SYNDROME IN PREMENOPAUSAL WOMEN" JOURNAL OF THE AMERICAN COLLEGE OF NUTRITION, AMERICAN COLLEGE OF NUTRION, WILMINGTON, NC, US, vol. 24, no. 6, December 2005 (2005-12), pages 486-493, XP009085779 ISSN: 0731-5724
- VILLANI R G ET AL: "L-Carnitine supplementation combined with aerobic training does not promote weight loss in moderately obese women" INTERNATIONAL JOURNAL OF SPORT NUTRITION 2000 UNITED STATES, vol. 10, no. 2, 2000, pages 199-207, XP009085992 ISSN: 1050-1606
- SEAMON M J ET AL: "EPHEDRA: YESTERDAY, DSHEA, AND TOMORROW - A TEN YEAR PERSPECTIVE ON THE DIETARY SUPPLEMENT HEALTH AND EDUCATION ACT OF 1994" JOURNAL OF HERBAL PHARMACOTHERAPY, HAWORTH HERBAL PRESS, BINGHAMTON, US, vol. 5, no. 3, 2005, pages 67-86, XP009085769 ISSN: 1522-8940
- ANONYMOUS: "X-Form plus mit Orangen Aroma" INTERNET ARTICLE, [Online] XP002440595 Retrieved from the Internet: URL:http://www.sevisana.ch/produkte/x_form _%20plus.php> [retrieved on 2007-07-03]
- ANONYMOUS: "Unterstützung der Fettverbrennung mit move bei bewegung" INTERNET ARTICLE, [Online] XP002440596 Retrieved from the Internet: URL:http://www.energy4life.ch/cms/front_co ntent.php?idcat=172> [retrieved on 2007-07-03]

## Description

The invention relates to a method for weight reduction in a mammal, comprising a diet that either reduces or lifts the inhibition of the fat metabolism by glucose, combined with an intake of a composition that contains one or several active agents that promote fat burning. The invention further relates to a method for the prevention of insulin resistance, for the prevention of Diabetes Mellitus type II and/or for the prevention of Syndrome X by the method in accordance with the invention for weight reduction in a mammal. The invention further relates to an invention of a composition for weight reduction in a mammal containing L-carnitine, a physiologically acceptable derivative and/or a salt thereof and Maté, as well as their use for treatment of obesity, for the prevention of insulin resistance, for the prevention of Diabetes Mellitus type II as well as for the prevention of Syndrome X.

As described in the literature, Syndrome X and especially Diabetes Mellitus type II are promoted by obesity and lack of exercise. Lack of exercise and obesity are especially connected to the increased risk of a future insulin resistance with a possible development of Diabetes Mellitus type II or of other clinical pictures of Syndrome X (Ernährungsmedizin und Diätetik, Heinrich Kasper, 10th Edition, Publishers Urban & Fischer, 2004).

Overweight can arise from too much energy input by excessive and continuous eating. Paucity of exercise and a high glycemic diet promote overweight. As a consequence of excessive and frequent eating, a deviation from the standard weight (overweight) can develop and related to it, an insulin resistance arising from a constant insulin secretion to an adjustment to the blood sugar. The basal metabolic rate falls with increasing age. If the energy intake is not reduced, then the retention of the eating habits may perhaps be the sole reason for a weight increase. If these correlations are not broken, then the Syndrome X and in particular Diabetes Mellitus type II is furthered and the clinical pictures of Syndrome X (metabolic syndrome) such as hyperlipoproteinemia and hypertension could finally set in. Epidemiological studies show that Diabetes Mellitus type II is appearing in increasingly younger people as a result of malnourishment and paucity of exercise, which finally leads to a moderate overweight (body mass index (BMI) 25-30 or obesity (adipose, BMI>30) in an increasingly younger levels of population.

A number of methods for the reduction of weight are known, such as diets that reduce the intake of food and/or energy, surgical intervention such as, for example, stomach reduction procedures or therapies with active agents, as for example appetite suppressants. All these approaches have disadvantages in that they do not aim at sustainability and the weight increases more rapidly than before after a diet or a treatment with an appetite suppressant according to the well-known yoyo effect and often during the diet or treatment period a feeling of being indisposed, weakness as well as gluttonous attacks appears.

The object the invention therefore is to provide a method for weight reduction in a mammal which does not possess the aforesaid disadvantages, does not have any side effect and with which a continuous weight reduction over many weeks or months can be achieved.

In accordance with the invention, this object has been achieved by the method according to Claim 1. The subject-matter of the invention is thus a method for weight reduction in a mammal, comprising a diet that either reduces or lifts inhibition of the fat metabolism by glucose, combined with an intake of a composition that contains one or several active agents that promote fat burning. Another object of the invention is a method for the prevention of insulin resistance, Diabetes Mellitus type II and/or for the prevention of Syndrome X by the method in accordance with the invention for weight reduction in a mammal. The invention further comprises a composition for weight reduction in a mammal comprising L-Carnitine, a physiologically acceptable derivative and/or a salt thereof, and Maté, as well as its use for weight reduction, for treatment of obesity, for the prevention of insulin resistance, for the prevention of Diabetes Mellitus type II as well as for the prevention of Syndrome X according to claims 11 and 12.

Under mammals, humans as well as other mammalians are understood herein, such as for example pets like dogs or cats, as also other mammalians such as horses or cattle. The method is suitably used in humans, preferably in moderate overweight humans (BMI 25-30), more preferably in obese people (adipose, BMI>30).

The inhibition of the fat metabolism depends on the glucose level in the blood. Intake of sugar or high-glycemic food raises the glucose level in the blood, which leads to an increased intake of glucose by the body cells such as the cells of the fat tissue, through the secretion of insulin. Frequent intake of sugar or high-glycemic food and the thereby caused continuous secretion of insulin and the associated glucose intake by these cells, can lead to a prolonged inhibition of the fat metabolism.

By inhibition of the fat metabolism by glucose as referred herein, the inhibition of the transport of fatty acids into the mitochondria is to be understood, in particular the inhibition of the transport of fatty acids into the mitochondria by the inhibition of the concerned enzymes such as the carnitine palmitoyltransferase I, the carnitine translocase and/or the carnitine palmitoyltransferase II by glucose or by the metabolic products of glucose such as malonyl-CoA.

By sweetening agents as referred herein, those substances are understood that are used to sweeten food item such as sugar, sweeteners and sugar substitutes. By sugar, general carbohydrates are to be understood, as for example, oligo-, di-, or monosaccharides like maltodextrin, maltotriose, saccharose, fructose, glucose, ribose or maltose, which can also be modified, such as for example fructose-1,6-diphosphate. By sweeteners, sweetening agents are to be understood that have an intensive sweet taste and no or very few calories as for example acesulfame, aspartame, cyclamate, saccharine, stevioside, thaumatin, neohesperidin DC and their salts as for example their sodium salts. Sugar substitutes have fewer calories than sugar but are also less sweet. Sugar substitutes are for example, sugar alcohols such as sorbitol, isomaltol, mannitol and xylitol.

By food as referred herein, solid and fluid food items are understood. Hereunder, fall all the food items that a mammal can take in orally or in any other form. By low-glycemic food, such food is understood that preferably contains carbohydrates which give up glucose into the blood as little as possible and as slow as possible during digestion.

The diet in accordance with the invention that reduces or lifts the inhibition of the fat metabolism consists in taking in no food in between principal meals that increases the glucose levels in the blood. Preferably, no food containing calories is taken at all between principal meals. Food that increases the glucose levels in the blood is usually glucose or general sugar containing food items. However, between principal meals, food items, which do not increase the blood glucose levels such as for example, salads can be taken. More preferably between principal meals, only beverages that do not increase the blood glucose levels like water, mineral water or tea without sugar are taken, whereby preferably these fluids do not contain calories. Especially, water or tea without sugar is taken between principal meals.

The principal meals suitably consist of low-glycemic food, as for example fruits and vegetables with plenty of fiber as well as lowest possible glycemic sugars as are present, for example, in wholemeal pasta. The principal meals consist preferably of food items with a Glycemic Index (GI) of < 55% as for example whole grain rice, dry fruits, pulses, fruits, boiled potatoes, vegetables, salads, nuts, milk, yogurt. The level of glucose in the blood is kept as low as possible by the low-glycemic food. Consequently very few metabolic products of glucose such as for example malonyl-CoA appear which can inhibit the transport of fatty acids into the mitochondria by the inhibition of the carnitine palmitoyltransferase I. The amount and the composition of the food should be balanced and should contain all the nutrients essential for the mammal such as vitamins and aminoacids and are fitted to suit individually the gender, age, body weight, the basal metabolic rate and the work metabolism corresponding to the physical activity. Also, in particular, the distribution of the food over morning, noon and evening should be adjusted accordingly; otherwise a weight reduction will be difficult. Evening is preferably the meal with the lowest calorie intake. The customary three principal meals during a day (24 hours) are suitably taken between 6 and 9 o'clock in the mornings, between 11 and 15 o'clock at noon and between 17 and 20 o'clock in the evening, whereby preferably between three to six hours, more preferably between four to six hours, no food is taken between the principal meals that increases the glucose levels in the blood, whereby preferably no calorie containing food is taken during this period.

Suitably, the energy balance of the diet in accordance with the invention is balanced out or negative. A balanced out energy balance is achieved when the energy content of the food corresponds to the number of calories that are necessary to sustain the existing body weight. A negative energy balance is achieved when the energy content of the food is less than the number of calories that are necessary to sustain the existing body weight. The number of calories that are necessary to sustain the existing body weight depends on the gender, age, body weight, the base metabolic rate as well as the work metabolism corresponding to the physical activity of a mammal. Preferably, the energy balance of the diet in accordance with the inventiont is negative.

Normally, the number of calories that are necessary to sustain the existing body weight in a person is the sum of the resting metabolic rate (1 kcal per kilogram body weight and hour) and the work metabolism, whereby the work metabolism is calculated from the basal metabolic rate multiplied by the corresponding factors for each particular physical activity (e.g. 1.5 for light physical activity). The energy content of the food for a negative energy balance is preferably less than 100 to 80 %, more preferably less than 80 to 60 %, especially less than 60 to 40 % of the number of calories that are necessary to sustain the existing body weight. The base metabolic rate or the resting metabolic rate is suitably measured in order to eliminate individual fluctuations, wherein the resting metabolic rate can also be calculated.

It was observed that the application of the diet according to the invention by itself leads to a weight reduction. In all the test persons, however, side effects such as weakness and/or indisposition as well as gluttonous attacks were reported. Surprisingly, with the intake of a composition that contains one or several active agents that promote fat burning, the said side effects cease. This is due to the fact that with the intake of the composition that contains one or several active agents which promote fat burning, utilizable energy is made available and the fat burning is accelerated. A test person reported at the start of the reduction cycle on changing over to the diet in accordance with the invention combined with the simultaneous intake of a composition that contains one or several active agents which promote fat burning, that after 2-3 days, the feeling of indisposition or gluttonousness were no longer to be felt and the diet could be well adhered to. Different persons of varying ages and sex and having different BMIs have tried out this combined method and the results show the causative link between application and adherence of the method and weight loss. If the diet was not adhered to, then even on an additional intake of L-canitine, no weight loss was reported.

The changeover to the diet in accordance with the invention as well as the intake of the composition is effectively started simultaneously, i.e., right from the first day of the changeover to the diet in accordance with the invention, the composition that contains one or several active agents which promote fat burning is taken.

Normally, the diet and the intake of the composition are continued for a period of 1 to 300 days, preferably for a period of 1 to 200 days, more preferably for a period of 7 to 100 days, most preferably for a period of 10 to 70 days. The diet and the intake of the composition can be continued as well as till a BMI of 20-25 or the targeted BMI is achieved.

The method in accordance with the invention is suitably supported by additional actions such as measures against lack of exercise as well as a sufficient fluid intake. These additional measures have a positive effect on the weight reduction wherein the rate of the body weight reduction per unit time can be enhanced. The adequate fluid intake should be 2-3 1 per day, preferably 21 per day.

By a composition that contains one or several active agents that promote fat burning, a composition is hereby understood, which on intake increases the oxidative breakdown of fatty acids per unit time in comparison with the oxidative breakdown of fatty acids per unit time without the intake of the composition. Usually, the composition which promote fat burning, increase on intake as well the basal metabolic rate. The composition that contains one or several active agents that promote fat burning is usually taken 1-3 times per day, preferably 1-2 times per day, wherein the one-time intake is taken more preferably in the morning, a twice a day intake, is taken more preferably in the morning and afternoon, a thrice a day intake, is taken more preferably in the morning, noon and evening. More preferably, the composition is not taken on an empty stomach but during or after the intake of a meal. The composition is effectively administered orally. Possible dosage forms are for example powder, tablets with and without, preferably however without coating, for example as compressed tablets, dragees, chewable dosage forms such as chewing gum or bars or fluid dosage forms for example as beverages, e.g. dissolved in water. Preferable dosage forms are powder, tablets with and without, preferably however without coating for example as compressed tablets, dragees, or fluid dosage forms for example as beverages, e.g. dissolved in water, more preferable are powder and liquid dosage forms, for example as beverages, e.g. dissolved in water. Preferably, the composition in accordance with the invention is taken orally, dissolved in an aqueous solution.

Normally the composition that promotes fat burning contains L-carnitine, genistein or other active agents that promote fat burning, in therapeutically effective amounts. Preferably, the composition that promotes fat burning contains L-carnitine, a physiologically acceptable derivative and/or a salt thereof as active agent. L-carnitine base ((3R)-hydroxy-4-(trimethylammonium)-butyrat), L-carnitine-L-tartrat, acetyl-L-carnitine, propionyl-L-carnitine, valeroyl-L-carnitine, isovaleroyl-L-carnitine, L-carnitine-magnesium-citrate, L-carnitin-fumarate or other known derivates and/or salts of L-carnitine are used suitably as L-carnitine, a physiologically acceptable derivative and/or salts thereof. L-carnitine-L-tartrate, acetyl-L-carnitine and/or L-carnitine base are preferred. More preferred is L-carnitine-L-tartrate. Also mixtures of the said L-carnitine can be used, however only one L-carnitine is preferably used at a time.

If the composition contains L-carnitine, a physiologically acceptable derivative and/or salt thereof, then this is preferably in an amount of 10-10000 mg, preferably in an amount of 200-5000 mg, more preferably in an amount of 200-2000 mg, especially in an amount of 300-1000 mg related to the amount of L-carnitine base in the composition. These quantities are based on a 70 kg body weight of the mammal. For a body weight deviating from this value, the quantity is to be appropriately adjusted.

Surprisingly it was found that with the intake of a composition that contains one or several active agents that promote fat burning and in addition one or several active agents that reduce the breakdown of fat to fatty acids in the intestines, the efficiency of the method according to the invention can be markedly increased as opposed to an intake of a composition that contains only one or several active agents which promote fat burning. Therefore, the composition contains, in a preferred embodiment in addition to one or several active agents that promote fat burning, one or several active agents that reduce the breakdown of fat to fatty acids in the intestines. Active agents that reduce the breakdown of fat to fatty acids in the intestines are herein active agents that inhibit enzymes which catalyse the breakdown of the fats (tricylglycerines) to fatty acids and glycerines. Active agents that reduce the breakdown of fats to fatty acids in the intestines are for example Maté that is normally derived from the plant *Ilex paraguariesis* and is commercially available, for example, from Frutarom Switzerland Ltd. Maté encompasses the leaves and twigs as well as all extracts from the leaves and twigs of the Ilex paraguariensis plant, preferably the plant *Ilex paraguarinesis* St.Hil as well as the active agents contained in it. Maté is preferably used as extract from the leaves of the plant *Ilex paraguariensis,* more preferably as ethanolic extract, in particular as ethanolic extract in powder form such as the commercially available extract EFLA® 920 Green Maté (Frutarom Switzerland Ltd). Maté is present in the composition suitably in an amount of 10-10000 mg, preferably in an amount of 300-3000 mg, more preferably in an amount of 200-2000 mg, referred to the amount of the ethanolic extract in powder form as contained in EFLA® 920 Green Maté. These amounts are based on a 70 kg body weight of the mammal. For a body weight deviating from this value, the quantity is to be appropriately adjusted.

Other active agents that reduce the breakdown of fat to fatty acids in the intestines are Green Tea extracts such as TEAVIGO^{™} (DSM, Netherlands) or the Green tea extract EFLA® 942 (Frutarom Switzerland Ltd). Green Tea extracts such as TEAVIGO^{™} (DSM, Netherlands), which are used as the preferred Green tea extracts, or EFLA® 942 (Frutarom Switzerland Ltd.) are contained in the composition suitably in an amount in the range of 10-5000 mg, preferably in an amount in the range of 50-1000 mg, more preferably in an amount of 50-300 mg. Maté is preferably used as the active agent that reduces the breakdown of fat to fatty acids in the intestines.

In addition, one or several active agents can be included in the composition that suppress the appetite such as SLIMALUMA^{™}, an extract from the Cactus Caralluma Fimbriata (commercially available from Giellepi chemicals, Srl), and/or one or more active agents that lower the glucose levels in the blood such as (3-O-methyl-D-chiro-inositol) that is preferred, D-Chiro-inositol (both commercially available for example from Amicogen Inc) or Fenulife (commercially available from Gieleppi chemicals, Srl) or coffee bean extract (Oryza Oil & Fat Chemical Co Ltd.). As well micro-nutrients such as vitamins in concentrated form as provided e.g. by Juice Plus+® (commercially available from NSA, Inc.) can be included in the composition of the present invention. These additional active agents are normally included in therapeutically effective quantities. The therapeutically effective amount of these active agents can vary depending on the body weight and metabolism. A "therapeutically effective amount" as referred herein is an amount effective to ameliorate or prevent the symptoms, or prolong the survival of the subject being treated. Determination of a therapeutically effective amount is well within the capabilities of those skilled in the art. For systemic administration, a therapeutically effective amount or dose can be estimated initially from in vitro assays. For example, a dose can be formulated in animal models to achieve a circulating concentration range that includes the IC50 as determined in cell culture. Such information can be used to more accurately determine useful doses in humans. Initial doses can also be estimated from in vivo data, e.g. animal models, using techniques that are well known in the art. One ordinarily skill in the art could readily optimize administration to humans based on animal data and will, of course, depend on the subject being treated, on the subject's weight, the severity of the disorder and the manner of administration.

Furthermore the composition can include additionally one or several sweetening agents as well as optionally in addition one or several flavouring agents and optionally one or several acidifying agents and optionally one or more colouring agents. As sweetening agents, suitably one or several sugars and/or one or several sweeteners and/or one or more sugar substitutes are used. Preferably one or several sugars and/or one or several sweeteners, more preferably one or several sugars and one or several sweeteners are used. As sugars, oligosaccharides like maltodextrin and maltotriose, as well as fructose, fructose-1,6-diphosphate, glucose, maltose are preferred, more preferred are oligosaccharides, in particular maltodextrin. Flavouring agents that can be used are for example lemon flavour, grape fruit flavour, apricot flavour or orange flavour. Acidifying agents that can be used are acidifying agents that are usually added to food items like citric acid or its salts. As flavouring agents, natural flavouring agents derived from the corresponding fruits as well as chemically synthesized, artificial flavouring agents can be used. As colouring agents, colouring agents that are commonly added to food items can be used like riboflavin or β-carotene.

If L-carnitine is used as the active agent that promotes fat burning, then the sweetening agent, flavouring agent, acidifying agents and colouring agent taken totally are present at least in a ratio (weight) of 0.1-0.5 to 1, preferably in a ratio of 0.25 -0.35 to 1, more preferably in a ratio of about 0.3 to 1 related to the amount of L-carnitine base in the composition.

It is known that Syndrome X and in particular Diabetes Mellitus II as well as insulin resistance are promoted by obesity and that through a weight reduction, especially the risk of a future insulin resistance and a resulting possible development of Diabetes Mellitus type II or other clinical pictures of Syndrome X are lowered.

Another object of the invention is thus a method for the prevention of insulin resistance; for the prevention of diabetes mellitus type II and/or for the prevention of Syndrome X through weight reduction in a mammal by the method for weight reduction described above in accordance with the invention.

A further object of the invention is a composition for weight reduction in a mammal containing L-carnitine, a physiologically acceptable derivative and/or salt thereof, and Maté. As L-canitine, a physiologically acceptable derivative and/or salt thereof, the L-carnitines described above are suitably used. L-carnitine-L-tartrate, acetyl-L-carnitine and/or L-carnitine base are preferred. More preferred is L-carnitine-L-tartrate. Mixtures of the said L-carnitines can also be used, however only one L-carnitine is preferably used at a time. The aforedescribed Maté is used as Maté. Maté is preferably used as extract from the leaves of the plant *Ilex paraguariensis,* more preferably as ethanolic extract, in particular as ethanolic extract in powder form such as the commercially available extract EFLA® 920 Green Maté.

Normally the composition contains an amount of 10-10000 mg of L-carnitine related to the amount of L-carnitine base and an amount of Maté of 10-10000 mg , related to the amount of ethanolic extract in powder form as contained in EFLA® 920 Green Maté. Preferred is an amount of 200-5000 mg, more preferably an amount of 200-2000 mg, in particular an amount of 300-1000 mg L-carnitine related to the amount of L-carnitine base. Maté is preferably used in an amount of 100-3000 mg, more preferably in an amount of 200-2000 mg, related to the amount of the ethanolic extract in powder form as contained in ELFA® 920 Green Maté. The composition is presented suitably in a dosage form for oral administration selected from powder, tablets, dragees, chewing gums, bars or beverages as described above.

The composition in accordance with the invention can suitably include the above said active agents such as green tea extract like TEAVIGO^{™} (DSM Netherlands) or ELFA® 942 (Frutarom Switzerland Ltd.) and/or natural products which suppress the appetite like SLIMALUMA^{™} and/or one or more active agents which lower the blood-glucose levels such as Pinitol (3-O-methyl-D-chiro-inisitol) that is preferred, D-Chiro-inositol (both commercially available for example from Amicogen Inc) or Fenulife (commercially available from Gieleppi chemicals, Srl) or coffee bean extract (Oryza Oil & Fat Chemical Co Ltd.). It can also include in addition to L-carnitine, another active agent, which promotes fat burning like Genistein. Furthermore the composition can additionally include one or several sweetening agents as well as optionally one or several flavouring agents and optionally one or several acidifying agents and optionally one or several colouring agents as defined above. Preferably it further comprises green tea extracts. As well preferred the composition comprises additionally one or several sweetening agents as well as optionally in addition one or several flavouring agents and optionally one or several acidifying agents and optionally one or several colouring agents.

Especially preferred is a composition comprising of L-carnitine, Maté, one or several sweetening agents as well as additionally one or more flavouring agents and optionally one or several acidifying agents and optionally one or several colouring agents. Also, specially preferred is a composition comprising of L-carnitine, Maté, green tea extract, one or several sweetening agents as well as additionally one or several flavouring agents and optionally one or several acidifying agents and optionally one or several colouring agents. Sweetening agent, flavouring agent, acidifying agent and colouring agent taken totally are present effectively in a ratio (weight) of 0.1- 0.5 to 1, preferably in a ratio of 0.25 -0.35 to 1, more preferably in a ratio of about 0.3 to 1 related to the amount of L-carnitine base in the composition.

The composition is suitably present in the form of a retail package. Under this presentation form are present also such retail packages with several compositions in accordance with the invention, optionally spatially separated from one another in identical and/or different dosages which allow for an exact dosing. The retail package contains suitably one or several normally air and watertight bags made for instance from polythene or aluminium, whereby preferably aluminium bags are used in which the composition in accordance with the invention is filled with the desired dosage.

A further object of the present invention is the composition in accordance with the invention for the treatment of obesity as well as the use of the composition in accordance with the invention for the manufacture of a medicament for weight reduction in a mammal in combination with a diet that either reduces or lifts the inhibition of the fat metabolism by glucose as well as the use of the composition in accordance with the invention for the manufacture of a medicament for the treatment of obesity in combination with a diet that either reduces or lifts the inhibition of the fat metabolism by glucose.

A further object of the present invention is the composition in accordance with the invention for the prevention of insulin resistance and/or for the prevention of Diabetes Mellitus type II and/or for the prevention of Syndrome X as well as the use of the composition in accordance with the invention for the manufacture of a medicament for the prevention of insulin resistance and/or for the prevention of Diabetes Mellitus type II and/or for the prevention of Syndrome X in combination with a diet that either reduces or lifts the inhibition of the fat metabolism by glucose

The present invention is explained in more detail with the help of the following examples.

### Examples

### Example 1 (not within the scope of the invention)

Person 1 (36 years), had a BMI of 25 and a starting weight of 72.5 kg on day 0, before using the method. Person 1 has taken his meals at the following times: Breakfast between 6 and 8 o'clock, lunch at 12 o'clock and evening meal at 6 to 8 o'clock. Between meals, no nourishment was taken except fluids. Fluids during the daytime (approx. 2 1) were water, morning's coffee and fresh juices, rarely wine during meals and now and then coffee without sugar. Sugar and chocolate were not taken by the person 1 during the whole time period of 29 days. Right from the beginning, every morning, before breakfast, following composition containing 1000 mg L-carnitine (related to L-carnitine base)

| | mg |
|---|---|
| L-carnitine-L-tatrate | 1470 |
| Maltodextrin | 10 |
| Sodium cyclamate | 30 |
| Sodium citrate | 9 |
| Sodium saccharine | 4 |
| Citric acid | 2 |
| Natural lemon flavour | 15 |
| Total | 1540 |
| **L-carnitine content (related to L-carnitine base)** | **1000** |

was taken throughout the total time period of 29 days. Person 1 had no problem in changing over to the diet and supplemented the method with 1-2 hours of fast walking per day as well as a light jogging 3 x in the week (30-45 min,). Throughout the time period of 29 days, no incidence of indisposition, no weakness and no gluttonous attacks were reported by Person 1. The calculated daily calorie requirement for Person 1 amounted to 2558 kcal (variance depending on the day 2400-2800 kcal.) The calculated daily mean energy content of the food consumed amounted to 1200 kcal (variance depending on the day 900 - 1500 kcal). The calculated mean energy content of weight loss amounted to 1590 kcal per day. The targeted weight reduction is made clear in Table 1.

**Table 1**

| Days | Weight |
|---|---|
| 0 | 72.5 |
| 7 | 70.2 |
| 15 | 68 |
| 22 | 67 |
| 29 | 66.5 |

### Example 2

Person 2 (37 years), had a BMI of 25.2 and a starting weight of 68.7 kg on day 0, before using the method. Person 2 had taken no nourishment between meals except fluids over a time period of 63 days. Fluids during the daytime (approx. 2 1) were water (an average of 2 dl per hour) and now and then coffee without sugar. Person 2 has taken his meals at the following times: breakfast 7.30, meal at 12 o'clock (mostly vegetables), dinner 6 o'clock (uncooked vegetarian food). Chocolates or other sweets were not taken by Person 2 during the whole time period of 63 days. From the beginning, the composition containing L-carnitine mentioned in example 1 was taken every morning with the breakfast over the complete time period of 63 days. Person 2 evinced 2 to 3 days of trouble keeping away from taking in nourishment between meals. After that, no problems like indisposition, weakness or gluttonous attacks were reported by person 2 over the remaining period of the 63 days. Fluid intake and physical activity were within the frame as before the use of the method, in the second phase of use, the physical activity was restricted. The calculated daily calorie requirement for person 2 amounted to 2324 kcal (variance depending on the day 2100-2500 kcal.) The calculated daily mean energy content of the food consumed amounted to 1300 kcal (variance depending on the day 1100 - 1600 kcal). The calculated mean energy content of weight loss amounted to 820 kcal per day. The targeted weight reduction is made clear in Table 2.

**Table 2**

| Days | Weight |
|---|---|
| 0 | 68.7 |
| 2 | 68.4 |
| 4 | 67.5 |
| 7 | 67.1 |
| 19 | 66.8 |
| 21 | 66 |
| 24 | 67.5 |
| 26 | 66.8 |
| 28 | 65.9 |
| 33 | 65.6 |
| 63 | 62 |

### Example 3

Person 3 (50 years), had a BMI of 30.8 and a starting weight of 78.8 kg on day 0, before using the method. Person 3 had taken no nourishment between meals except fluids over a time period of 42 days; fluids during the daytime (approx. 2 1) water or tea and now and then coffee without sugar. Person 3 has taken his meals at the following times: breakfast, 7 o'clock (bread, apple) principal meal at 12 o'clock (largely vegetables, supplements, and meat), dinner 6 o'clock (bread, cheese, and fruit). Person 3 during the whole time period of 42 days did not take chocolates or other sweets. From the beginning, the composition containing L-carnitine mentioned in Example 1 was taken every morning with the breakfast over the complete time period of 42 days (mornings, before the morning meal). Physical activity was increased and 0.5 h walking per day within the framework of the method was introduced. Throughout the time period of 42 days, no incidence of indisposition, no weakness and no gluttonous attacks were reported by Person 3. The calculated daily calorie requirement for Person 3 amounted to 2565 kcal (variance depending on the day 2300-2700 kcal.) The calculated daily mean energy content of the food consumed amounted to 1600 kcal (variance depending on the day 1400 - 1800 kcal). The calculated mean energy content of weight loss amounted to 880 kcal per day.

The targeted weight reduction is made clear in Table 3.

**Table 3**

| Days | Weight |
|---|---|
| 0 | 78.8 |
| 7 | 78 |
| 14 | 78 |
| 21 | 76.9 |
| 28 | 76.3 |
| 35 | 75.9 |
| 42 | 74 |

### Example 3A

Person 3 has continued with the method as described in Example 3 up to a time period of 140 days. The calculated daily calorie requirement for Person 3 over the total time period of 140 days amounted to 2565 kcal (variance depending on the day 2200-2700 kcal.) The calculated daily mean energy content of the food consumed amounted to 1600 kcal (variance depending on the day 1200 -1800 kcal). The calculated mean energy content of weight loss amounted to 462 kcal per day respectively 0.42 kg / week weight loss. The calculated BMI was reduced to 27.5. The targeted weight reduction is made clear in Table 3A.

**Table 3A**

| Days | Weight | BMI |
|---|---|---|
| 0 | 78.8 | 30.8 |
| 7 | 78 | 30.5 |
| 14 | 78 | 30.5 |
| 21 | 76.9 | 30.0 |
| 28 | 76.3 | 29.8 |
| 35 | 75.9 | 29.6 |
| 42 | 74 | 28.9 |
| 75 | 72 | 28.1 |
| 126 | 70.7 | 27.6 |
| 140 | 70.4 | 27.5 |

### Example 4

Person 4 (45 years), had a BMI of 25.5 and a starting weight of 71.0 kg on day 0, before using the method. Person 4 had taken no nourishment between meals except fluids over a time period of 11 days; fluids during the daytime (approx. 2 1) water or tea and now and then coffee without sugar. Person 4 has taken his meals at the following times: breakfast, 7 o'clock (bread, orange juice, 1 yogurt) principal meal at 12 o'clock (largely vegetables, supplements, meat), dinner 6 o'clock (bread, cheese, fruit). Person 4, during the whole time period of 11 I days, left out chocolates or other sweets. From the beginning, every morning with the breakfast, 750 mg (corresponding to 500 mg L-carnitine related to L-carnitine base) of the composition mentioned in example was taken along with 600 mg EFLA® 920 green Maté (Frutarom Switzerland Ltd.) dissolved in water or orange juice throughout the time period of 11 days (mornings before, during or after the morning meal). The physical activity was 0.5 hours walking per day and was neither increased nor decreased as against before the onset of the method. Throughout the time period of 11 days, no incidence of indisposition, no weakness and no gluttonous attacks were reported by Person 4. The calculated daily calorie requirement of Person 4 amounted to 2433 kcal (variance depending on the day 2200-2600 kcal.) The calculated daily mean energy content of the food consumed amounted to 1600 kcal (variance depending on the day 1300 - 1900 kcal). The calculated mean energy content of weight loss amounted to 1400 kcal per day. The targeted weight reduction is made clear in Table 4.

**Table 4**

| Days | Weight |
|---|---|
| 0 | 71 |
| 2 | 70 |
| 5 | 69.5 |
| 11 | 69 |

### Example 5 (not within the scope of the invention)

Person 5 (15.5 years), had a BMI of 32.2 and a starting weight of 99.8 kg on day 0, before using the method. Person 5 was surveyed by a medical doctor throughout the whole period of 278 days. Person 5 has taken his meals at the following times: Breakfast between 6 and 8 o'clock, lunch at 12 o'clock and evening meal at 6 to 8 o'clock. Between meals, no nourishment was taken except fluids. Fluids during the daytime (approx. 2 1) were water, morning's coffee and a glas of fruit juice. Sugar and chocolate were reduced to the minimum taken by the person 5 during the whole time period of 278 days. Right from the beginning, every morning, before breakfast, the composition containing 1000 mg L-carnitine (related to L-carnitine base) as mentioned in example 1 was taken throughout the total time period of 278 days. Person 5 had no problem in changing over to the diet and supplemented the method with 0.5 to 1 hours of swimming once or twice a week or cycling (1-2 times in the week, 30-60 min). Throughout the time period of 278 days, no incidence of indisposition, no weakness and no gluttonous attacks were reported by Person 5. The calculated daily calorie requirement for Person 5 amounted to 2756 kcal (variance depending on the day 2200-3000 kcal.) The calculated daily mean energy content of the food consumed amounted to 1200 kcal (variance depending on the day 600 - 1600 kcal). The calculated mean energy content of weight loss amounted to 687 kcal per day. The targeted weight reduction is made clear in Table 5. Blood values were measured twice after approx. 100 days and approx. 170 days. The triglyceride values were 1.6 mmol/l and 0.8 mmol/l, respectively. HDL (High density lipoprotein) values were reported as 1.16 and 1.10 mmol/l, respectively, and cholesterol values were reported to be 2.7 and 3.5 mol/l, respectively. The values reported show that the triglyceride values could be lowered during the weight reduction time.

**Table 5**

| days | weight | BMI |
|---|---|---|
| 0 | 99.8 | 32.2 |
| 7 | 99.2 | 32.0 |
| 14 | 98 | 31.6 |
| 21 | 96.8 | 31.3 |
| 28 | 95.4 | 30.8 |
| 30 | 95 | 30.7 |
| 92 | 89.3 | 28.8 |
| 126 | 85 | 27.4 |
| 167 | 80.2 | 25.9 |
| 185 | 79.4 | 25.3 |
| 238 | 76.9 | 24.5 |
| 278 | 75 | 23.9 |

### Example 6

Person 6 (46 years), had a BMI of 25.1 and a starting weight of 71.0 kg on day 0 before using the method. Person 6 had taken no nourishment between meals except fluids over a time period of 9 days; fluids during the daytime (approx. 2 1) water or tea or coffee without sugar. Person 6 has taken his meals at the following times: breakfast, 7 o'clock (bread, apple) lunch at 12 o'clock (largely vegetables, supplements, and meat), dinner 6.30 o'clock (bread, cheese, and fruit). From the beginning, every morning with the breakfast, 750 mg L-Carnitine L-tartrate (corresponding to 500 mg L-carnitine related to L-carnitine base) mixed with 600 mg EFLA® 920 green Maté and 150 mg grean tea EFLA® 942 (Frutarom Switzerland Ltd.) dissolved in water throughout the time period of 9 days (mornings before, during or after the morning meal) were consumed. The physical activity was approx. 0.5 hours walking per day and was neither increased nor decreased as against before the onset of the method. Throughout the time period of 9 days, no incidence of indisposition, no weakness and no gluttonous attacks were reported by Person 6. The calculated daily calorie requirement of Person 6 amounted to 2300 kcal (variance depending on the day 2200-2400 kcal.) The calculated daily mean energy content of the food consumed amounted to 1880 kcal (variance depending on the day 1500 - 2200 kcal). The calculated mean energy content of weight loss amounted to 1027 kcal per day. The measured weight values are shown in Table 6.

**Table 6**

| days | weight |
|---|---|
| 0 | 71 |
| 5 | 69.5 |
| 8 | 70 |
| 9 | 69.8 |

## Claims

1. A method for weight reduction in a mammal, comprising a diet, wherein no food containing calories is taken in between all principal meals combined with an Intake of a composition that contains one or several active agents that promote fat burning wherein the active agent that promotes fat burning is L-carnitine, a physiologically acceptable derivative and/or a salt thereof, with the provisio that the method for weight reduction is not therapeutically indicated.

2. The method according to claim 1, wherein the diet and the intake of the composition are continued for a period of 1 to 300 days.

3. The method according to any of claims 1 to 2, wherein the composition is taken 1 to 3 times per day.

4. The method according to claim 1, wherein the L-carnitine is L-carnitine-tartrate, acetyl- L-carnitine and/or L-carnitine base.

5. The method according to claim 4, wherein L-carnitine, a physiologically acceptable derivative and/or salt thereof is present in the composition in an amount of 10 to 10000 mg.

6. The method according to any of claims 1 to 5, wherein the composition contains additionally one or several active agents that reduce the breakdown of fat to fatty acids in the intestines.

7. The method according to claim 6 , wherein the active agent that reduces the breakdown of fat to fatty acids in the intestines is Mate.

8. The method according to claim 7, wherein Mate is present in the composition in an amount of 10 to 10000 mg.

9. The method according to any of claims 1 to 8, wherein the composition contains additionally one or several active agents that suppress the appetite and/or one or several active agents that lower the glucose levels In the blood.

10. The method according to any of claims 1 to 9, wherein the composition contains additionally one or several sweetening agents as well as optionally in addition one or several flavouring agents and optionally one or several acidifying agents and optionally one or several colouring agents.

11. Composition comprising L-carnitine, a physiologically acceptable derivative and/or salt thereof, In combination with a diet wherein no food containing calories is taken in between all principal meals for use for the treatment of obesity.

12. Composition comprising L-carnitine, a physiologically acceptable derivative and/or salt thereof, in combination with a diet wherein no food containing calories is taken In between all principal meals for use for the prevention of insulin resistance and/or for the prevention of Diabetes Mellitus type II and/or for the prevention of Syndrome X.

## Patentansprüche

1. Ein Verfahren zur Gewichtsreduktion in einem Säuger umfassend eine Diät, wobei kein kalorienhaltiges Nahrungsmittel zwischen allen Hauptmahlzeiten zu sich genommen wird, wobei zusätzlich zu der Diät eine Zusammensetzung eingenommen wird, welche ein oder mehrere aktive Wirkstoffe enthält, die die Fettverbrennung fördern, wobei der aktive Wirkstoff, der die Fettverbrennung fördert, L-Carnitin ist, ein physiologisch verträgliches Derivat und/oder ein Salz davon, mit der Massgabe, dass das Verfahren zur Gewichtsreduktion nicht therapeutisch angezeigt ist.

2. Das Verfahren gemäss Anspruch 1, wobei die Diät und die Einnahme der Zusammensetzung in einer Periode von 1 bis 300 Tagen erfolgt.

3. Das Verfahren gemäss einem der Ansprüche 1 bis 2, wobei die Zusammensetzung 1 bis 3 mal pro Tag eingenommen wird.

4. Das Verfahren gemäss Anspruch 1, wobei das L-Carnitin L-Carnitin-tartrate, Acetyl-L-carnitin und/oder L-Carnitin Base ist.

5. Das Verfahren gemäss Anspruch 4, wobei L-Carnitin, ein physiologisch verträgliches Derivat und/oder ein Salz davon in der Zusammensetzung in einer Menge von 10 bis 10000mg vorhanden ist.

6. Das Verfahren gemäss einem der Ansprüche 1 bis 5, wobei die Zusammensetzung zusätzlich eine oder mehrere aktive Wirkstoffe enthält, die den Abbau von Fett zu Fettsäuren im Darm reduzieren.

7. Das Verfahren gemäss Anspruch 6, wobei der aktive Wirkstoff, der den Abbau von Fett zu Fettsäuren im Darm reduziert. Mate ist.

8. Das Verfahren gemäss Anspruch 7, wobei Mate in der Zusammensetzung in einer Menge von 10 bis 10000mg vorhanden ist.

9. Das Verfahren gemäss einem der Ansprüche 1 bis 8, wobei die Zusammensetzung zusätzlich eine oder mehrere aktive Wirkstoffe enthält, die den Appetit unterdrücken und/oder eine oder mehrere aktive Wirkstoffe, die den Blutzuckersplegel senken.

10. Das Verfahren gemäss einem der Ansprüche 1 bis 9, wobei die Zusammensetzung zusätzlich eine oder mehrere Süssungsmittel enthält sowie gegebenenfalls zusätzlich eine oder mehrere Aromastoffe und gegebenenfalls eine oder mehrere Säuerungsmittel und gegebenenfalls eine oder mehrere Farbstoffe.

11. Zusammensetzung umfassend L-Carnitin, ein physiologisch verträgliches Derivat und/oder ein Salz davon in Kombination mit einer Diät, wobei kein kalorienhaltiges Nahrungsmittel zwischen allen Hauptmahlzeiten zu sich genommen wird, zur Anwendung zur Behandlung von Fettleibigkeit.

12. Zusammensetzung umfassend L-Carnitin, ein physiologisch verträgliches Derivat und/oder ein Salz davon in Kombination mit einer Diät, wobei kein kalorienhaltiges Nahrungsmittel zwischen allen Hauptmahlzeiten zu sich genommen wird, zur Anwendung zur Vorbeugung von Insulinresistenz und/oder zur Vorbeugung von Diabetes Mellltus Typ II und/oder zur Vorbeugung von Syndrom X.

## Revendications

1. Une méthode pour la réduction du poids d'un mammifère, comprenant un régime alimentaire, dans lequel aucun aliment contenant des calories n'est pris entre les repas principaux combiné avec un apport d'une composition qui contient un ou plusieurs agents actifs qui favorisent la combustion des graisses dans lequel l'agent actif qui favorise la combustion des graisses est la L-carnitine, un dérivé acceptable sur le plan physiologique et/ou un sel de celui-ci, à condition que la méthode de réduction du poids ne soit pas contrindiquée sur le plan thérapeutique.

2. La méthode selon la revendication 1, dans lequel le régime alimentaire et l'apport de la composition sont poursuivis pendant une période de 1 à 300 jours.

3. La méthode selon l'une quelconque des revendications 1 à 2, dans laquelle la composition est prise de 1 à 3 fois par jour.

4. La méthode selon la revendication 1, dans laquelle la L-carnitine est la L-carnitine tartrate, l'acétyl-L-carnitine et/ou la base de L-camitine.

5. La méthode selon la revendication 4, dans laquelle la L-carnitine, un dérivé acceptable sur le plan physiologique et/ou un sel de celui-ci est présent dans la composition en quantité de 10 à 10000 mg.

6. La méthode selon l'une quelconque des revendications 1 à 5, dans laquelle la composition contient en plus un ou plusieurs agents actifs qui permettent de réduire la dégradation des graisses en acides gras dans les intestins.

7. La méthode selon la revendication 6, dans laquelle l'agent actif qui réduit la dégradation des graisses en acides gras dans les intestins est le Mate.

8. La méthode selon la revendication 7, dans laquelle le Mate est présent dans la composition en quantité de 10 à 10000 mg.

9. La méthode selon l'une quelconque des revendications 1 à 8, dans laquelle la composition contient en plus un ou plusieurs agents actifs qui suppriment l'appétit et/ou un ou plusieurs agents actifs qui font baisser le taux de glucose dans le sang.

10. La méthode selon l'une quelconque des revendications 1 à 9, dans laquelle la composition contient en plus un ou plusieurs édulcorants ainsi que, optionnellement en plus un ou plusieurs agents aromatisants et optionnellement un ou plusieurs agents acidifiants et optionnellement un ou plusieurs agents de coloration.

11. Une composition comprenant la L-carnitine, un dérivé acceptable sur le plan physiologique et/ou un sel de celui-ci, en combinaison avec un régime alimentaire dans lequel aucun aliment contenant des calories n'est pris entre les repas principaux pour son utilisation dans le traitement de l'obésité.

12. Une composition comprenant la L-carnitlne, un dérivé acceptable sur le plan physiologique et/ou un sel de celui-ci, en combinaison avec un régime alimentaire dans lequel aucun aliment contenant des calories n'est pris entre les repas principaux pour son utilisation dans la prévention de la résistance à l'insuline et/ou dans la prévention du diabète sucré de type II et/ou dans la prévention du syndrome X.
